# EUROPÄISCHE PATENTSCHRIFT

(11) **EP 1 437 673 B1**
(45) Veröffentlichungstag und Bekanntmachung des Hinweises auf die Patenterteilung: **29.07.2009**
(21) Anmeldenummer: 03090419.7
(22) Anmeldetag: 03.12.2003
(51) Int. Cl.: G06F 19/00, G06F 17/30, G01N 33/68, C12Q 1/04

(54) **Verfahren zur Identifizierung von Mikroorganismen mittels Massenspektrometrie**
Method for the identification of microorganisms by mass spectrometry
Méthode d'identification de microorganismes par la spectrométrie de masse

(30) Priorität: 07.01.2003 DE 10300743
(43) Veröffentlichungstag der Anmeldung: 14.07.2004
(73) Patentinhaber: AnagnosTec Gesellschaft für Analytische Biochemie und Diagnostik mbH, 14476 Potsdam OT Golm (DE)
(72) Erfinder: Kallow, Wibke, 12307 Berlin (DE); Dieckmann, Ralf, 10707 Berlin (DE); Erhard, Marcel, 14476 Potsdam OT Golm (DE); Sauermann, Stefan, 14469 Potsdam (DE)
(74) Vertreter: Reinstädler, Diane

(56) Entgegenhaltungen:
- EP-A- 1 253 622
- WO-A-00/29987
- GB-A- 2 187 035
- WILKES J G ET AL: "Defining and using microbial spectral databases" JOURNAL OF THE AMERICAN SOCIETY FOR MASS SPECTROMETRY, ELSEVIER SCIENCE INC., NEW YORK, NY, US, Bd. 13, Nr. 7, Juli 2002 (2002-07), Seiten 875-887, XP004371094 ISSN: 1044-0305
- VAIDYANATHAN S ET AL: "Flow-injection electrospray ionization mass spectrometry of crude cell extracts for high-throughput bacterial identification" JOURNAL OF THE AMERICAN SOCIETY FOR MASS SPECTROMETRY, ELSEVIER SCIENCE INC., NEW YORK, NY, US, Bd. 13, Nr. 2, Februar 2001 (2001-02), Seiten 118-128, XP004334676 ISSN: 1044-0305

## Beschreibung

Die Erfindung betrifft ein Verfahren zur Identifizierung von Mikroorganismen mittels Massenspektrometrie, insbesondere mittels Matrix-assisted-Laser-Desorption-Ionisation Time-of-Flight Massenspektrometrie (MALDI-TOF-MS).

Eine schnelle und zuverlässige Identifizierung von Mikroorganismen ist in verschiedenen Bereichen des Gesundheitswesens, beispielsweise der Diagnostik von Infektionen, sowie der Lebensmittelindustrie von entscheidender Bedeutung. Die traditionelle Identifizierung mittels des direkten Bakteriennachweises erfordert zunächst die Anzüchtung der zu identifizierenden Mikroorganismen aus einer Materialprobe. Anschließende mikroskopische und visuelle Untersuchungsverfahren dienen hauptsächlich der vorläufigen Orientierung über den Bakteriengehalt sowie der Mikromorphologie beziehungsweise Färbeeigenschaften der klinischen Untersuchungsprobe. Die Identifizierung von Isolaten bis zur Speziesebene erfordert nicht selten eine Subkultivierung zwecks der Gewinnung einer Reinkultur. Nach einem klassischen Identifizierungsverfahren der medizinischen Mikrobiologie, der so genannten "Bunten Reihe", werden unter Verwendung einer geeigneten Kombination von Differentialmedien spezifische Stoffwechselleistungen des zu identifizierenden Mikroorganismus erfasst. Hauptnachteil des mikrobiellen Verfahrens ist sein sehr hoher Zeitbedarf, insbesondere für die Kultivierung.

Modernere molekularbiologische Ansätze, wie die PCR-Methode (Polymerase-Kettenreaktion) und die 16S-rRNA-Methode, involvieren die genetische Analyse des zuvor isolierten Genoms beziehungsweise bestimmter Ribonukleinsäuren. Diese Verfahren haben wegen ihrer hohen Empfindlichkeit stark an Bedeutung gewonnen. Sie sind jedoch ebenso wie die mikrobiologische Charakterisierung mit einem erheblichen personellen und instrumentellen Aufwand belastet.

Ferner sind infrarotspektroskopische Verfahren bekannt, bei denen Schwingungsspektren intakter Zellen ("Fingerprintspektren") in FTIR-Spektrometern aufgezeichnet und mit einer Datenbank mit Schwingungsspektren bekannter Mikroorganismen abgeglichen wird. Diese noch junge Technik befindet sich noch in der Entwicklung und kann derzeit nur von speziell geschultem und erfahrenem Personal durchgeführt werden, so dass sich dieser Ansatz noch nicht in der Praxis durchsetzen konnte.

Mit der so genannten MALDI-TOF-Massenspektrometrie ist in den letzten Jahren ein Verfahren entwickelt worden, das im Gegensatz zu herkömmlichen massenspektrometrischen Verfahren auch der Analyse biologischer Makromoleküle zugänglich ist. Bei der MALDI-TOF-MS-Technik wird die zu untersuchende Probe mit einer meist kristallinen organischen Verbindung, der so genannten Matrix, auf eine Probenplatte gegeben, wobei die Probe in die Matrixkristalle eingebaut wird, und mit einem Laserstrahl in Wechselwirkung gebracht. Dabei werden einzelne Moleküle der Probe von dem Probenträger desorbiert und ionisiert. Anschließend werden die so erzeugten Ionen in einem elektrischen Feld beschleunigt und ihre Flugzeit (Time-of-Flight) bis zum Erreichen eines Detektors registriert. Da die Beschleunigung von der Masse eines ionisierten Moleküls abhängt, spiegeln die Flugzeiten die in der Probe vorhandenen Molekülmassen wider. Die MALDI-TOF-MS-Technik findet heutzutage hauptsächlich auf dem Gebiet der Proteinanalytik ("Proteomics") und bei der RNA- und DNA-Analytik Anwendung.

Es wurde zum Beispiel in der WO 98/09314 vorgeschlagen, die MALDI-TOF-Massenspekrometrie zur Identifizierung von Mikroorganismen in Analogie zu der oben beschriebenen infrarotspektroskopischen Fingerprintmethode einzusetzen. Dafür wird das MALDI-TOF-Massenspektrum eines Zellextraktes oder intakter Zellen des unbekannten Mikroorganismus mit den Spektren bekannter Organismen verglichen. Der Vergleich des Probenspektrums mit den Referenzspektren der Datenbank erfolgt in der Regel computergestützt mittels statistisch-mathematischer Algorithmen, die in so genannten Mustererkennungsverfahren entwickelt wurden. Die Massenspektren weisen mit wachsender verwandtschaftlicher Nähe der Mikroorganismen zunehmende Ähnlichkeit miteinander auf und die Wahrscheinlichkeit einer Wiederholung bestimmter Signale in den Massenspektren nimmt zu. Dies hat zur Folge, dass das Verfahren bereits bei übergeordneten Klassifizierungsniveaus, etwa bei Gattungen oder Familien, mit einer erhöhten Unsicherheit verbunden ist, wenn eine Zuordnung auf einem untergeordneter Niveau (zum Beispiel Stammniveau) fehlschlägt, beispielsweise weil kein Referenzspektrum des Stammes in der Datenbank vorhanden ist. Das Verfahren ist daher auf sehr umfangreiche Datenbanken mit einer Vielzahl repräsentativer Referenzspektren von bekannten Stämmen angewiesen. Hinzu kommt die Schwierigkeit, ein spektrales Grundrauschen von echten Signalen zu diskriminieren.

Aus GB 2 187 035 A ist ein Verfahren zur Identifizierung von Mikroorganismen mittels Massenspektroskopie bekannt, bei dem die zu untersuchende Probe zunächst durch einen Pyrolyseprozess in primäre Fragmente gespalten wird, die in einem Quadropolmassenspektrometer bestimmt werden. In einem zweiten Analyseschritt erfolgt eine weitere Fragmentierung und eine Aufnahme eines Massenspektrums dieser Sekundärfragmente.

WO 00/29987 A beschreibt ein Verfahren zur Bestimmung von Mikroorganismen mittels MALDI-TOF-MS, wobei ein Massenspektrum des zu bestimmenden Organismus aufgenommen und mit einer Datenbank verglichen wird, die Proteinmassen enthält. Die Proteinmassen werden aus Nukleotid- oder Aminosäuresequenzen vorausgesagt.

Aus Wilkes et al. (J. Am. Soc. Mass Spectr., Bd. 13 (7), 2002, S. 875-887) ist ein Verfahren bekannt, bei dem das Massenspektrum eines zu bestimmenden Mikroorganismus gemessen und nachfolgend mit einem geeigneten Algorithmus transformiert wird, um eine Kompatibilität mit einer vorhandenen Referenzdatenbank herzustellen. Dabei soll die Transformation Variationen des Massenspektrums ausgleichen, die durch unterschiedliche instrumentelle Bedingungen, Kultivierungsbedingungen und dergleichen verursacht werden.

Vaidyanathan et al. (J. Am. Soc. Mass Spectr., Bd. 13 (2), 2001, S. 118-128) beschreiben ein Verfahren zur Identifizierung von Bakterien, bei dem zellfreie Extrakte durch Tandem-MS untersucht werden. Dabei werden in einem ersten Schritt mittels ESI-MS die unfragmentierten Zellbestandteile untersucht und nachfolgend die einzelnen Komponenten weiter fragmentiert. Der eigentliche Spektrenabgleich erfolgt durch Hauptkomponenten- und anschließender Clusteranalyse.

Aus den DE 100 38 694 A und EP 1 253 622 A ist ein Verfahren zur Identifizierung von Mikroorganismen mittels MALDI-TOF-MS bekannt, bei dem das Probenspektrum des unbekannten Mikroorganismus nicht mit Originalmassenspektren ("natürlichen Massenspektren") abgeglichen wird, sondern mit so genannten synthetischen Referenzspektren. Dabei handelt es sich um Massenspektren, die aus einer reduzierten Anzahl von für den jeweiligen Mikroorganismus charakteristischen Signalen zusammengefasst sind. Diese charakteristischen Signale umfassen vorzugsweise solche, die bestimmten molekularen Bestandteilen der Zelle zugeordnet werden konnten und/oder die durch visuelle oder rechnergestützte Analyse als spezifisch selektiert wurden. Obwohl dieses Verfahren aufgrund der Sektion der Signale und Beschränkung des Spektrenvergleichs auf die selektierten Signale eine deutlich verbesserte Zuverlässigkeit gegenüber dem herkömmlichen Abgleich mit Originalspektren aufweist, hat sich die Unterscheidung sehr ähnlicher Organismen in Einzelfällen als schwierig erwiesen. Dies gilt insbesondere für eng verwandte Organismen.

Aufgabe der vorliegenden Erfindung ist es daher, das massenspektroskopische Verfahren zur Identifizierung von Mikroorganismen in Hinblick auf eine noch weiter erhöhte Zuverlässigkeit weiterzuentwickeln. Es soll ferner eine für das Verfahren geeignete Datenbank zur Verfügung gestellt werden.

Diese Aufgabe wird durch ein Verfahren mit den Merkmalen des Anspruchs 1 gelöst.

Das erfindungsgemäße Verfahren zur Identifizierung von Mikroorganismen mittels Massenspektrometrie, sieht vor, dass
(a) eine Datenbank verwendet wird, umfassend
   - synthetische Referenzspektren bekannter Mikroorganismen, welche durch Zusammenfassung einer gegenüber natürlichen Massenspektren reduzierten Anzahl von für den jeweiligen Mikroorganismus spezifischen Signalen gebildet werden, sowie
   - Differenzspektren, welche durch Verrechnung jeweils zweier synthetischer Referenzspektren der bekannten Mikroorganismen gebildet werden,
(b) in einem ersten Analyseschritt eine Ähnlichkeitsanalyse eines Probenmassenspektrums eines zu identifizierenden Mikroorganismus mit den in der Datenbank enthaltenen synthetischen Referenzspektren durchgeführt wird und
(c) in einem zweiten Analyseschritt eine Ähnlichkeitsanalyse des Probenspektrums mit wenigstens einem Teil der in der Datenbank enthaltenen Differenzspektren durchgeführt wird.

Das erfindungsgemäße Verfahren unterscheidet sich demnach von dem bekannten Verfahren im Wesentlichen durch einen zweiten Analyseschritt, bei dem das Probenspektrum mit Differenzspektren, die in noch näher zu beschreibender Weise aus den synthetischen Referenzspektren berechnet werden, verglichen wird. Dieser zusätzliche Schritt bewirkt eine signifikante Steigerung der Sicherheit des Verfahrens und ermöglicht insbesondere auch die Unterscheidung stark verwandter Organismen.

Die Differenzspektren werden vorzugsweise durch Subtraktion zweier synthetischer Referenzspektren voneinander gebildet, wobei Signale, die in beiden der miteinander verrechneten Referenzspektren vorhanden sind, unabhängig von ihren Intensitäten vollständig eliminiert werden. Es kann weiterhin vorgesehen sein, Signale, die nach der Subtraktion in einem Differenzspektrum eines bekannten Mikroorganismus verbleiben, noch weiter zu selektieren und/oder zu gewichten. Dies kann etwa dadurch geschehen, dass ein Abgleich der verbleibenden Signale mit den natürlichen Massenspektren (Originalspektren) dieses Mikroorganismus und/oder mit den natürlichen Massenspektren des mit diesem verrechneten Mikroorganismus erfolgt. Insbesondere kann hierfür die Häufigkeit und/oder Intensität des Signals in den "eigenen" natürlichen Massenspektren überprüft werden. Darüber hinaus ist es von Vorteil, auch die Spezifität des Signals, das heißt die Häufigkeit und/oder Intensität des Signals in den natürlichen Spektren ("Fremdspektren") desjenigen Mikroorganismus zu analysieren, dessen synthetisches Referenzspektrum mit dem des betreffenden Mikroorganismus verrechnet wurde.

Um beispielsweise ein Differenzspektrum für *Escherichia coli* zu generieren, das die Diskriminierung von den schwer unterscheidbaren *Shigellen* erleichtert, wird das synthetische Referenzspektrum von *Shigella sonnei* von dem von E. *coli* subtrahiert, wobei nur Signale übrig bleiben, die im synthetischen Referenzspektrum von E. *coli* nicht aber in dem von *S. sonnei* vorhanden sind. Anschließend wird jedes verbleibende Signal hinsichtlich seiner Häufigkeit und/oder Intensität mit den Originalspektren von *E. coli* und hinsichtlich seiner Häufigkeit und/oder Intensität in den Originalspektren von *S. sonnei* untersucht. Ein Signal, das zwar häufig in den eigenen Originalspektren ist, aber unspezifisch ist, weil es auch in den Fremdspektren von *Shigella* auftaucht, wird entweder ganz aus dem Differenzspektrum eliminiert oder für den zweiten Analyseschritt mit einer relativ geringen Gewichtung versehen. Auf diese Weise entstehen Differenzspektren, die eine sehr sichere Unterscheidung eines Mikroorganismus von einem bestimmten anderen erlauben.

Es ist weiterhin besonders bevorzugt vorgesehen, als Ergebnis des ersten Analyseschrittes eine Anzahl von mit dem Probenspektrum ähnlichen synthetischen Referenzspektren zu ermitteln und die Ähnlichkeitsanalyse des zweiten Schrittes nur mit denjenigen Differenzspektren durchzuführen, die aus der Verrechnung der als ähnlich ermittelten synthetischen Referenzspektren erhalten wurden. Somit erfolgt in dem ersten Schritt eine Eingrenzung der in Frage kommenden Mikroorganismen, so dass im anschließenden zweiten Schritt nur noch ein Abgleich mit den Differenzspektren der als ähnlich ermittelten Organismen erfolgt. Hierdurch wird nicht nur ein enormer Zeitvorteil erlangt, sondern es werden auch Zufallstreffer reduziert, die aus nicht identischen Proteinen mit zufällig übereinstimmenden Massen resultieren. In diesem Zusammenhang kann es ferner sinnvoll sein, eine Datenbank zu verwenden, die von vornherein nur Differenzspektren von einander ähnlichen Mikroorganismen enthält.

Es ist vorgesehen, dass als Referenzspektren synthetische Massenspektren verwendet werden, die durch Zusammenfassung einer gegenüber "natürlichen" (nicht reduzierten) Massenspektren reduzierten Anzahl von für den jeweiligen Mikroorganismus spezifischen Signalen erzeugt werden. Durch die Reduzierung der Referenzspektren auf eine verhältnismäßig geringe Anzahl charakteristischer Signale, wird eine erhebliche Daten- und Informationsreduktion erreicht. Durch die Datenreduktion wird nicht nur Speicherplatz eingespart, sondern auch ein zeitlicher Aufwand für Datenübertragungen, so dass das Verfahren prinzipiell auch für eine Anwendung über vernetzte Datenverarbeitungsanlagen (zum Beispiel Internet) geeignet ist. Darüber hinaus ermöglicht die Informationsreduktion der Referenzspektren der Datenbank eine deutliche Beschleunigung der Ähnlichkeitsanalyse des Massenspektrums des zu identifizierenden Mikroorganismus mit den Referenzspektren, da die Analyse sich nunmehr auf einen Vergleich der in den Referenzspektren enthaltenen Signale beschränken kann.

Verglichen mit herkömmlichen Verfahren, in denen ein Probenspektrum des zu identifizierenden Organismus mit "natürlichen" Referenzspektren abgeglichen wird, ist eine Zuverlässigkeit des erfindungsgemäßen Verfahrens, das heißt die Wahrscheinlichkeit, eine korrekte Zuordnung des unbekannten Organismus zu treffen, deutlich erhöht. Hierin ist der bedeutendeste Vorteil des Verfahrens zu sehen. Die erhöhte Zuverlässigkeit ist durch die hohe Konzentrierung spezifischer Informationen in den synthetischen Referenzspektren zu erklären, die nicht durch Signale geringer Signifikanz oder Rauschen überdeckt wird. Auch bei einem möglichen Versagen bei einer Zuordnung auf Stammebene einer Probe, etwa weil kein Referenzspektrum dieses Stammes in der Datenbank verfügbar ist, liefert das Verfahren zuverlässige Identifizierungen auf übergeordneten Klassifizierungsebenen, beispielsweise auf Gattungs- oder Artenebene. Die Empfindlichkeit der erfindungsgemäßen Vorgehensweise wird auch nicht durch Unterschiede in verschiedenen Spektren beeinträchtigt, welche beispielsweise durch unterschiedliche Kultivierungsbedingungen, unterschiedliche Zellstadien oder abweichende Signal-Rauschverhältnisse verursacht werden.

Ein weiterer Vorteil des Verfahrens stellt die geringe erforderliche Probenmenge des zu identifizierenden Mikroorganismus dar, die in kürzeren Kultivierungszeiten erhältlich ist. Ferner können auch Mischkulturen analysiert werden, so dass auf die Anzucht von Reinkulturen verzichtet werden kann.

Die in den synthetischen Referenzspektren enthaltenen Signale können in zwei Kategorien unterschieden werden. Eine besonders vorteilhafte Ausgestaltung der Erfindung sieht vor, dass die Signale eines Referenzspektrums mindestens ein identifiziertes Signal umfassen, welches einem charakterisierten molekularen Zellbestandteil des jeweiligen Mikroorganismus eindeutig zugeordnet wurde. Zur Identifizierung eines Mikroorganismus geeignete Zellbestandteile sind beispielsweise bestimmte Peptide, Proteine, Ribonukleinsäuren und/oder Lipide. Es hat sich insbesondere für Bakterien als vorteilhaft erwiesen, eine Signalzuordnung eines ribosomalen Proteins, insbesondere eines Proteins der großen Ribosomenuntereinheit, vorzunehmen. Für Bakterien sind dies Proteine der so genannten 50S-Untereinheit und bei Pilzen die 60S-Untereinheit. Proteine stellen einen Hauptbestandteil mikrobieller Zellen dar. Dies gilt insbesondere für Ribosomenproteine, die unabhängig von einem Entwicklungsstadium der Zelle, einem Nährstoffangebot oder sonstiger Kultivierungsbedingungen ständig präsent sind und somit zuverlässige Signale in den Massenspektren darstellen. Hinzu kommt, dass Aminosäuresequenzen analoger Proteine unterschiedlicher Spezies oder sogar unterschiedlicher Stämme sich zumindest geringfügig voneinander unterscheiden. Folglich weisen die analogen Proteine unterschiedliche Massen auf und eignen sich für deren Unterscheidung. So gelang den Erfindern beispielsweise erstmalig die Zuordnung von vier Massensignalen in dem Massenspektrum von *Escherichia coli* (m/z = 4365, 5381, 7276 und 5096) zu den Proteinen L36, L34 und L29 der großen 50S-Ribosomenuntereinheit beziehungsweise zu dem Protein S22 der kleinen 30S-Untereinheit. Diese Signale sind ständige Bestandteile in Massenspektren von *E. coli,* nicht jedoch vieler anderer Mikroorganismen. Sie sind daher besonders zur Identifizierung von *Escherichia coli* und zur Aufnahme in ein synthetisches Referenzspektrum für *E. coli* geeignet. Im Falle von Pilzen haben sich überdies ganz besonders Strukturproteine, insbesondere Hydrophobine zur Identifizierung bewährt.

In einer weiteren Ausbildung der vorliegenden Erfindung ist vorgesehen, dass die Signale eines synthetischen Referenzspektrums als zweite Signalkategorie mindestens ein empirisches Signal umfassen, welches durch Vergleich einer Vielzahl von Massenspektren bekannter Mikroorganismen als spezifisch für einen Mikroorganismus ermittelt wurde. Dabei handelt es sich um Signale, deren Ursprung, das heißt deren verursachende molekulare Zellkomponente, zwar nicht bekannt ist, die jedoch aufgrund bestimmter Kriterien als charakteristisch für einen Mikroorganismus gewertet werden. Vorzugsweise umfassen diese Kriterien eine vorgebbare Mindest-Häufigkeit für das Auftreten eines Signals in einer Anzahl von Massenspektren desselben Mikroorganismus sowie eine vorgebbare durchschnittliche Mindest-Intensität des Signals. Dabei sollte die Mindest-Häufigkeit mindestens 50 %, insbesondere mindestens 70 %, vorzugsweise mindestens 90 %, betragen. Zusätzlich wird die Spezifität des Signals überprüft, das heißt die Häufigkeit, mit der das Signal in den natürlichen Massenspektren anderer Mikroorganismen auftaucht, wobei das Auftreten in den Fremdspektren möglichst selten sein sollte. Die Ermittlung der empirischen Signale durch Vergleich gemessener Massenspektren kann visuell, vorzugsweise jedoch computergestützt, durchgeführt werden. Entsprechende Algorithmen (Mustererkennungsverfahren) sind bekannt und sollen hier nicht näher erläutert werden. Darüber hinaus ist es denkbar, auch die identifizierten Signale einer nachträglichen Kontrolle durch Anwendung dieser Kriterien zu unterziehen.

Die Anzahl der Signale eines Referenzspektrums kann in einem Rahmen von 1 bis 50 liegen und beträgt vorteilhafterweise 5 bis 30. In vielen Fällen hat sich insbesondere eine Anzahl von 10 bis 15 als ausreichend erwiesen. Es ist ferner bevorzugt vorgesehen, dass ein Signal eines synthetischen Referenzspektrums durch lediglich ein Koordinatenpaar repräsentiert wird. Dabei besteht das Koordinatenpaar aus einer Masse beziehungsweise einem Masse-Ladungs-Verhältnis als x-Koordinate einerseits und einer absoluten oder relativen Intensität als y-Koordinate andererseits. Verglichen mit "natürlichen" Massenspektren, die mehrere 1000 Datenpunkte enthalten, bedeutet dies eine erhebliche Datenreduktion.

Für den Abgleich eines Probespektrums mit den synthetischen Referenzspektren der Datenbank können vorteilhafterweise den einzelnen identifizierten und empirischen Signalen der Referenzspektren Gewichtungen entsprechend ihrer Signifikanz zugeordnet werden, wobei auf die oben genannten Kriterien, das heißt Häufigkeit/Intensität in den eigenen Spektren und in denen der anderen Referenzorganismen, zurückgegriffen werden kann. Wenn diese Gewichtung bereits auf Stufe der synthetischen Referenzspektren vorgenommen wird, kann auf eine entsprechende Selektion und/oder Gewichtung der Signale der Differenzspektren unter Umständen verzichtet werden.

Eine zur Durchführung des erfindungsgemäßen Verfahrens für die Identifizierung von Mikroorganismen geeignete Datenbank umfasst
(a) synthetische Referenzspektren bekannter Mikroorganismen, beinhaltend eine gegenüber natürlichen Massenspektren reduzierte Anzahl von für den jeweiligen Mikroorganismus spezifischen Signalen, sowie
(b) Differenzspektren, resultierend aus einer Verrechnung jeweils zweier synthetischer Referenzspektren der bekannten Mikroorganismen.

Darüber hinaus kann es von Vorteil sein, auch die natürlichen Referenzspektren (Originalspektren) in der Datenbank zu enthalten.

Das erfindungsgemäße Verfahren lässt sich besonders vorteilhaft mit der MALDI-TOF-Massenspektrometrie durchführen. Entsprechend sind alle verwendeten Massenspektren vorzugsweise MALDI-TOF-Massenspektren.

Die Erfindung wird nachfolgend in Ausführungsbeispielen anhand der zugehörigen Zeichnungen näher erläutert. Es zeigen:
- Figur 1: ein Ablaufschema des Verfahrens zur Erzeugung einer erfindungsgemäßen Datenbank;
- Figur 2: MALDI-TOF-Massenspektren (Originalspektren) von *Escherichia coli* und von isolierten Ribosomen aus *Escherichia coli*;
- Figuren 3 bis 8: synthetische Referenzspektren verschiedener Bakterien, insbesondere für zwei *Escherichia coli*-Stämme, zwei *Klebsiella-Spezies, Pseudomonas aeruginosa* und *Staphylococcus aureus;*
- Figur 9: ein synthetisches Referenzspektrum des Pilzes *Trichoderma reesei;*
- Figuren 10a-10d: eine Erzeugung zweier Differenzspektren;
- Figur 11: eine Struktur der erfindungsgemäßen Datenbank; und
- Figur 12: ein Ablaufschema des Verfahrens zur Identifizierung eines Mikroorganismus.

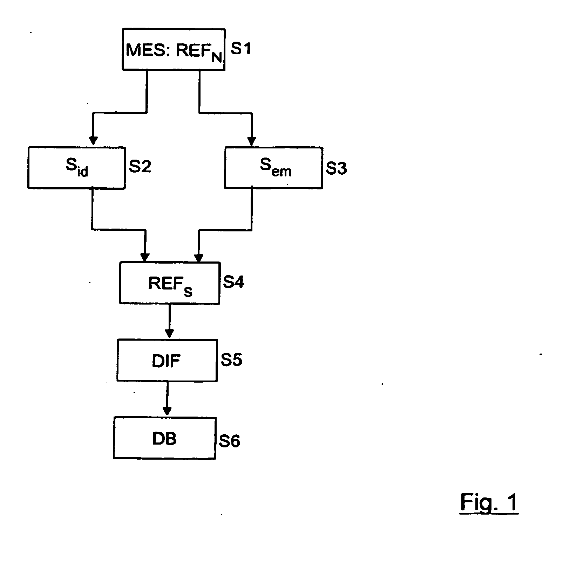

Figur 1 zeigt in einem Fließschema einen typischen erfindungsgemäßen Verfahrensablauf. In einem ersten Schritt S1 wird eine möglichst große Anzahl von natürlichen Referenzspektren REF_{N,} das heißt Originalspektren bekannter Mikroorganismen, gemessen. In diesem Zusammenhang werden im Folgenden einige Details zur Probenvorbereitung und zur Datenakquisition gegeben.

### Probenvorbereitung und MALDI-TOF-Datenakquisition

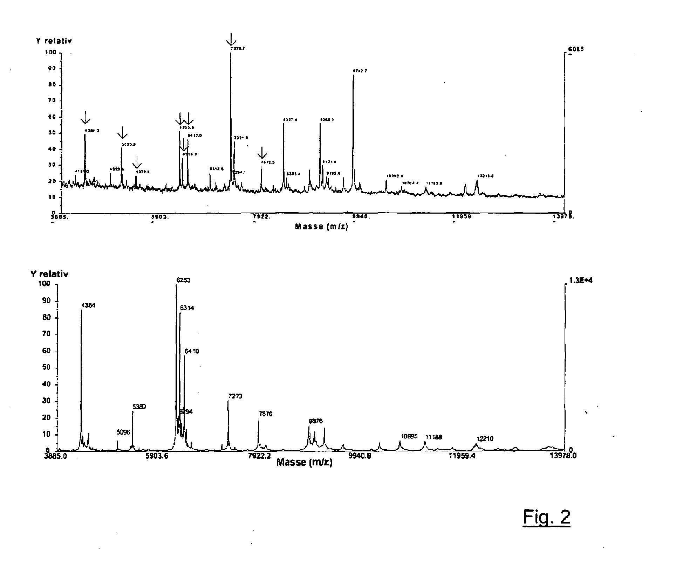

Für eine MALDI-TOF-Analyse werden zirka 5 bis 100 µg Nasszellen oder auch 5 bis 50 µg getrocknete Zellen eines bekannten oder zu identifizierenden Bakteriums oder Pilzes benötigt. Eine Vorbehandlung der Zellen, beispielsweise ein Zellaufschluss, ist nicht notwendig. Die Nasszellen können direkt von einer AGAR-Kultur mit einer sterilen Impföse auf eine auch Template genannte Probenplatte übertragen werden. Alternativ können auch abzentrifugierte Zellen einer Flüssigkultur verwendet werden. Anschließend werden die Zellen auf der Probenplatte mit 0,2 bis 1 *µ*l einer Matrixlösung vermischt. In Abwandlung von dieser Prozedur können die Nasszellen auch vor ihrer Übertragung auf die Probenplatte mit der Matrixlösung vermischt und als Suspension übertragen werden. Für die folgenden Messungen wurde eine Matrixlösung aus 100 mg/ml 2,5-Dihydroxybenzoesäure in einer Mischung aus 50 % Acetonitril und 50 % Wasser mit 3 % Trifluoressigsäure verwendet. Andere bekannte Matrixlösungen sind ebenfalls geeignet. Nach Trocknung der Probe, die mit einer Kristallbildung einhergeht, kann die Probe direkt einer MALDI-TOF-massenspektrometischen Analyse unterworfen werden. Für die vorliegenden Messungen wurde jeder Probenpunkt einer Probenplatte in einem herkömmlichen Massenspektrometer mit zirka 50 bis 300 Laserpulsen eines Stickstoff-Lasers mit einer Wellenlänge von 337 nm angeregt. Die Akquisition positiver Ionenmassenspektren erfolgte im Linearmessmodus in einem Massenbereich von 2000 bis 20000 m/z. Ein typisches Beispiel eines auf diese Weise erhaltenen positiven MALDI-TOF-Massenspektrums REF von *Escherichia coli* ist im oberen Teil der Figur 2 im Massenbereich von zirka m/z = 4000 bis 14000 dargestellt.

Im anschließenden Verfahrensschritt S2 (Figur 1) wird eine Zuordnung von Signalen (Peaks) zu bestimmten molekularen Zellbestandteilen durchgeführt. Eine mögliche Vorgehensweise, die auf bekannte Methoden der Biochemie und Molekularbiologie zurückgreift, wird im Folgenden am Beispiel von Ribosomenproteinen der großen Ribosomenuntereinheit kurz erläutert.

### Identifizierung unbekannter Signale

Ein Proteinextrakt einer Zellkultur wird mittels einer 2D-Gelelektrophorese aufgetrennt. In Frage kommende so genannte Proteinspots werden anschließend einem tryptischen Verdau, bei dem das Protein enzymatisch in kleine Proteinbruchstücke gespaltet wird, unterzogen. Sofern Antikörper gegen Ribosomen vorhanden sind, können die relevanten Proteinspots auch durch ein Immunoassay (zum Beispiel Western-Blot-Analyse) erkannt werden. Die durch den tryptischen Verdau gewonnenen Proteinbruchstücke werden dann mittels einer HPLC aufgetrennt und ansequenziert oder direkt einer so genannten Peptidmassen-Fingerprint-Identifizierung mit anschließender PSD (post source decay) unterzogen. Mit den auf diese Weise ermittelten Sequenzbruchstücken kann dann versucht werden, die Ribosomengene in einer Datenbank zu identifizieren. Sind entsprechende Gene in der Datenbank vorhanden, kann aus der gesamten Gensequenz die korrespondierende Proteinmasse ermittelt werden. Kann ein entsprechendes Gen in der Datenbank nicht gefunden werden, so muss mit bekannten Mitteln der Molekularbiologie, die hier nicht näher ausgeführt werden sollen, das gesamte Gen, welches für das entsprechende ribosomale Protein kodiert, isoliert und sequenziert werden. Ist die Gensequenz bekannt, folgt die Übersetzung in die Proteinsequenz und Ermittlung der theoretischen Proteinmasse. Eine Überprüfung dieser theoretischen Masse kann erfolgen, indem der entsprechende Proteinspot der 2D-Gelelektrophorese einer MALDI-TOF-Massenspektrometrie unterworfen wird. Bei Abweichungen von der theoretischen Proteinmasse können Modifikationen des Proteins durch MALDI-TOF-Analyse des tryptischen Verdaus festgestellt werden.

Zur Verdeutlichung ist in der Figur 2 im unteren Teil ein Massenspektrum des 70S-Ribosomens aus *Escherichia coli,* das mittels 2D-Gelelektrophorese isoliert wurde, dargestellt. Mit 70S wird der gesamte Ribosomen eines Proteins bezeichnet, der sich aus der großen 50S-Untereinheit und der kleinen 30S-Untereinheit zusammensetzt. Beide Untereinheiten bestehen ihrerseits aus einer Reihe von Proteinen, die mit dem Buchstaben L (für large) und dem Buchstaben S (für small) bezeichnet werden. Es ist ohne weiteres ersichtlich, dass eine Reihe der intensivsten Signale aus dem Spektrum von *Escherichia coli* ATCC 25922(vergleiche Figur 2 oberer Teil) sich auf ribosomale Proteine zurückführen lässt. Entsprechende Signale sind in der Abbildung mit Pfeilen gekennzeichnet. Den Erfindern ist es erstmalig gelungen, drei dieser Signale bestimmten Proteinen der großen 50S- und ein Signal einem Protein der kleinen 30S-Ribosomenuntereinheit zuzuordnen. Dabei handelt es sich um die Proteine L29, L34, L36 und S22 mit den Massen m/z = 7274, 5381, 4365 beziehungsweise 5096. Da insbesondere die Signale der großen Untereinheit mit einer hohen Zuverlässigkeit in den Massenspektren von *Escherichia coli* auftauchen, sind sie besonders zur Identifizierung geeignet. Sie wurden als identifizierte Signale S_{id} für das synthetische Referenzspektrum REF_{S} für *Escherichia coli* verwendet.

In einem alternativen oder zusätzlichen Schritt S3 (Figur 1) werden empirische Signale Sₑₘ aus den gemessenen Referenzspektren REF_{N} der bekannten Mikroorganismen ermittelt. Die Ermittlung der empirischen Signale Sₑₘ erfolgt durch Vergleich einer Vielzahl von Massenspektren REF_{N}, die von demselben Stamm aufgezeichnet wurden, untereinander sowie durch Vergleich mit denen von anderen Organismen. Dabei werden solche Signale als charakteristisch für einen Mikroorganismus ermittelt, die möglichst häufig in den Massenspektren desselben Organismus auftauchen und möglichst selten in den Massenspektren eines anderen. Für die Häufigkeit eines Auftretens eines geeigneten empirischen Signals Sₑₘ kann dabei ein Mindest-Wert, beispielsweise > 70 % bezogen auf alle Spektren eines Organismus, vorgegeben werden. Ebenso sollte ein solches Signal eine vorgebbare Mindest-Intensität besitzen, um seine Unterscheidung von dem Untergrundrauschen zu erleichtern. Die Ermittlung der empirischen Signale Sₑₘ im Schritt S3 kann zwar prinzipiell durch visuellen Spektrenvergleich durch den Anwender erfolgen. Es ist jedoch bevorzugt vorgesehen, diesen Schritt automatisiert mit Hilfe geeigneter Rechnerprogramme durchzuführen. Hier kommt etwa ein Algorithmus in Frage, welcher die gemessenen natürlichen Referenzspektren REF_{N} hinsichtlich der genannten Kriterien untersucht. Es sind jedoch auch, beispielsweise aus der Infrarotspektrometrie, computergestützte Verfahren bekannt, die unter Anwendung statistischer Algorithmen in der Lage sind, wiederkehrende Signale zu erkennen und herauszufiltern.

Die in den Schritten S2 und S3 ermittelten identifizierten und empirischen Signale S_{id}, Sₑₘ werden in einem anschließenden Schritt S4 zu synthetischen Referenzspektren REF_{S} zusammengefasst. Dabei wird für jeden bekannten Mikroorganismus ein Referenzspektrum REF_{S} erzeugt.

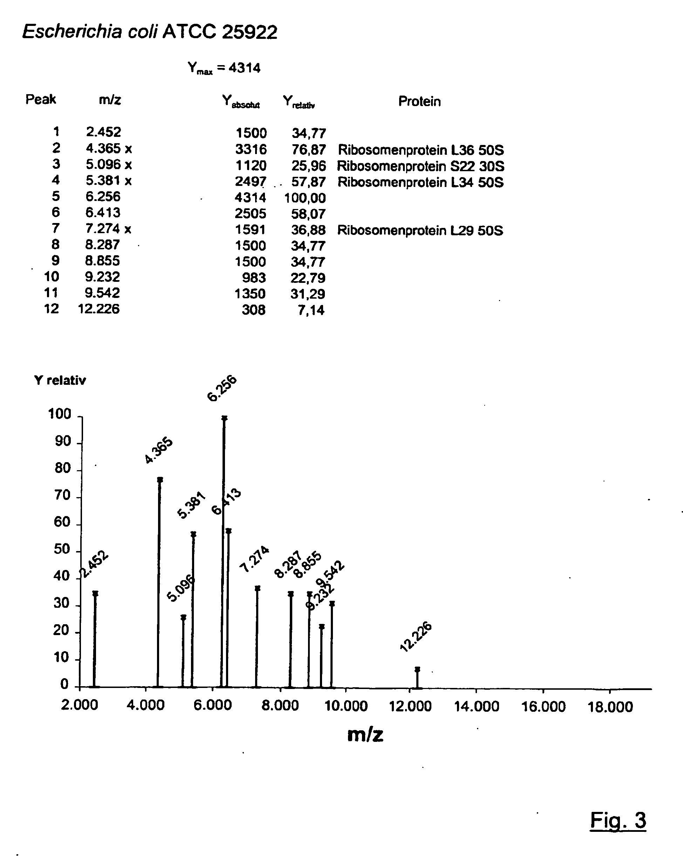

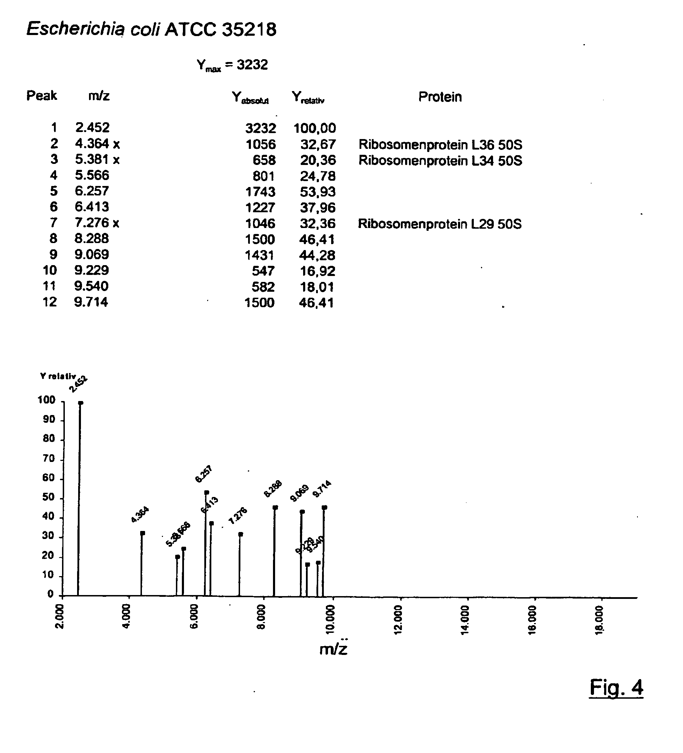

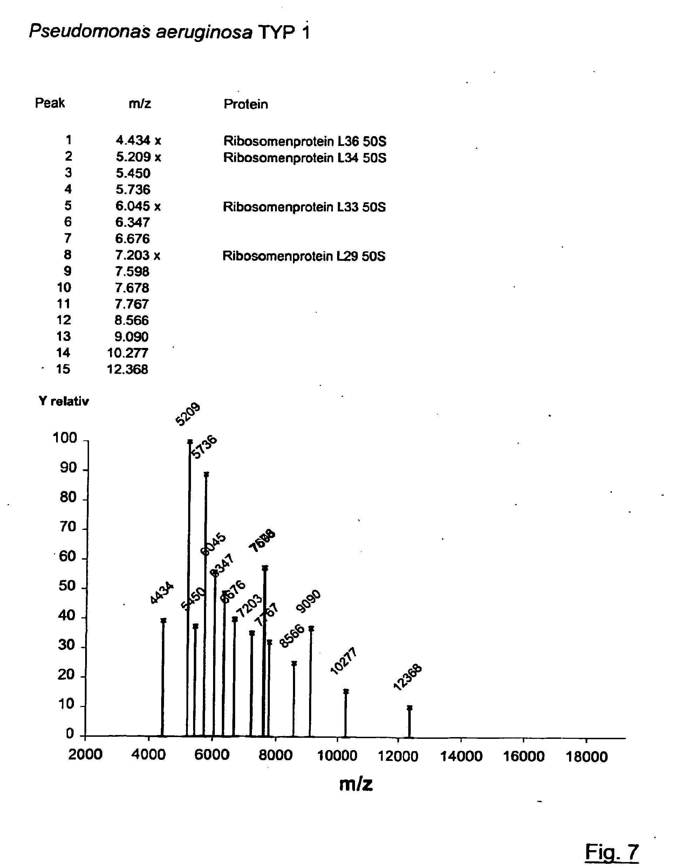

In den Figuren 3 bis 9 sind beispielhaft derartige synthetische Referenzspektren REF_{S} für zwei *Escherichia coli*-Stämme (ATCC 25922 und ATCC 35218), zwei *Klebsiella-Spezies -* nämlich *Klebsiella oxycoca* und *Klebsiella pneumoniae* -, für *Pseudomonas aeruginosa, Staphylococcus aureus* sowie für den Pilz *Trichoderma reesei* dargestellt. Dabei ist jeweils im oberen Teil jeder der Figuren 3 bis 9 das synthetische Referenzspektrum REF_{S} in Koordinatenform dargestellt (teilweise unter Verzicht der y-Koordinaten), während im unteren Teil jeweils die graphische Repräsentation in der typischen Form von Massenspektren abgebildet ist. Die synthetischen Referenzspektren REF_{S} umfassen in den gezeigten Beispielen zehn bis fünfzehn Signale S, im Falle des Pilzes T. *reesei* fünf. In den Referenzspektren REF_{S} von *Escherichia coli* (Figuren 3 und 4) und von *Pseudomonas aeruginosa* (Figur 7) sind die als Proteine der großen 50S-Ribosomenuntereinheit identifizierten Signale S_{id} markiert. Dabei konnten für *E*. *coli* und für *P. aeruginosa* jeweils die den Proteinen L29, L34 und L36 der großen Untereinheit zugehörigen Signale identifiziert werden sowie für *P. aeruginosa* zusätzlich das dem Protein L33 zugehörige Signal. Im Falle von *E. Coli* ATCC 25922 (Figur 3) konnte ferner ein Signal dem Protein S22 der kleinen 30S-Ribosomenuntereinheit zugeordnet werden.

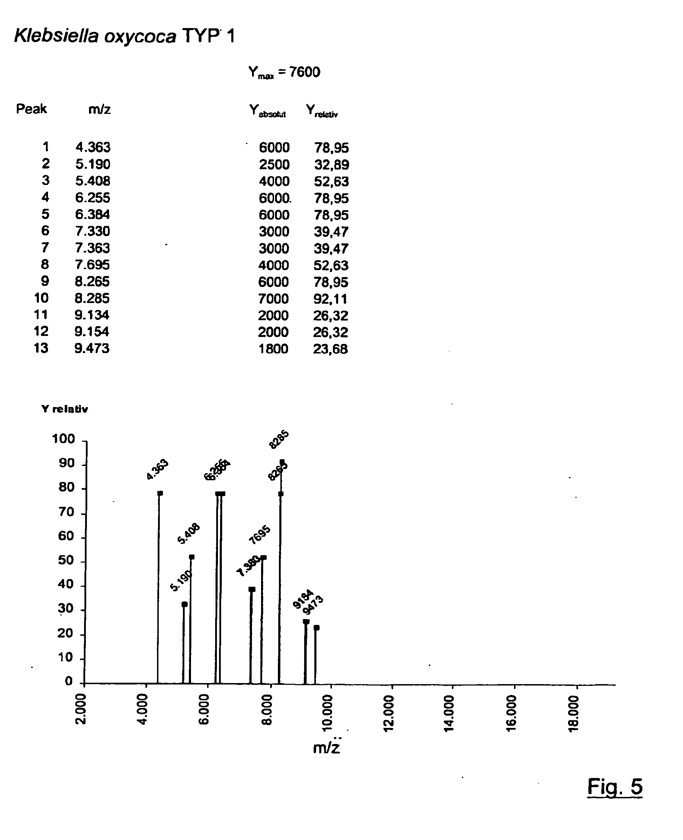

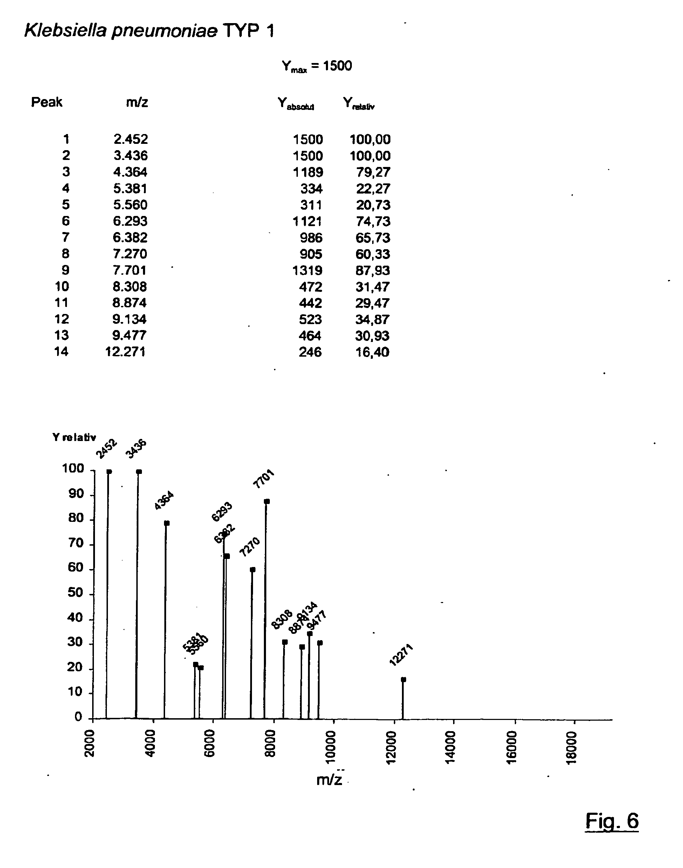

Die Unterscheidung der beiden *Escherichia coli*-Stämme untereinander (Figuren 3 und 4) wird hauptsächlich durch solche empirische Signale Sₑₘ ermöglicht, die nur bei einem von beiden Stämmen beobachtet werden. Ein Vergleich der in den Figuren 3 und 4 gezeigten synthetischen Referenzspektren REF_{S} zeigt, dass das erfindungsgemäße Verfahren empfindlich genug ist, um eine Unterscheidung von Mikroorganismen sogar auf Stammniveau zu ermöglichen. Daneben wird auch eine Unterscheidung zwischen *Escherichia coli* einerseits und *Klebsiella*-Spezies (Figuren 5 und 6) andererseits ermöglicht.

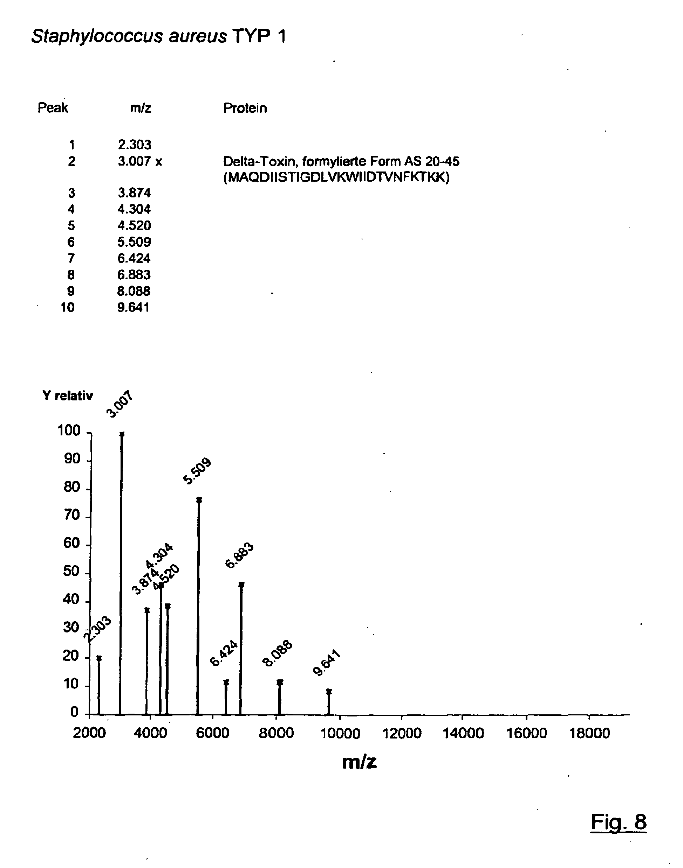

Das in Figur 8 dargestellte Beispiel von *Staphylococcus* aureus zeigt, dass auch andere als Ribosomenproteine identifiziert und zur Bestimmung des Organismus herangezogen werden können. In diesem Fall konnte ein Signal einem formylierten Peptidfragment des Delta-Toxins (Delta-Hemolysin) zugeordnet werden, das äußerst charakteristisch für dieses Bakterium ist.

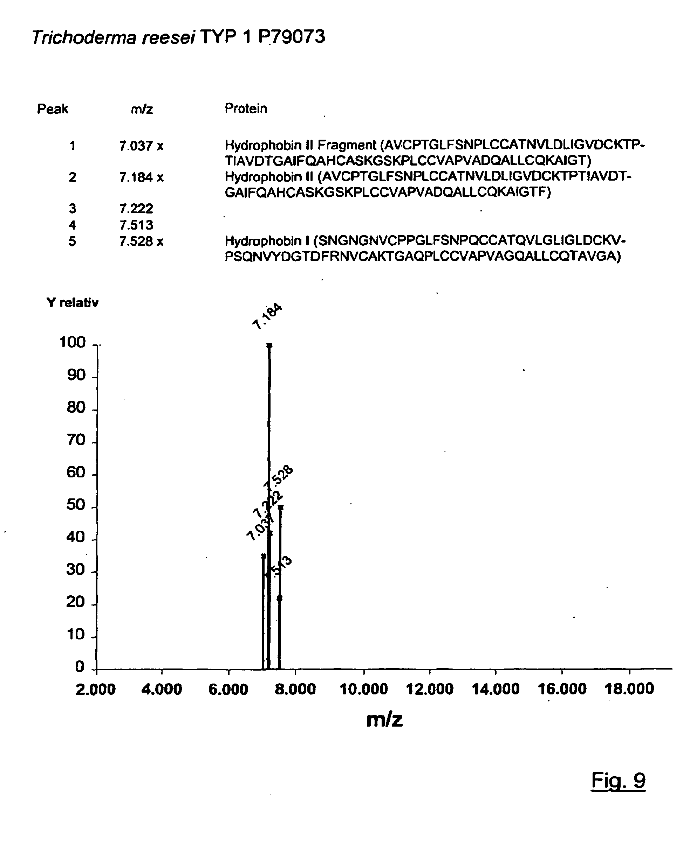

Im Falle von Pilzen haben sich neben ribosomalen Proteinen insbesondere auch die hydrophoben Strukturproteine Hydrophobine als vorteilhaft zur Identifizierung des Mikroorganismus erwiesen. So konnten für den Pilz *Trichoderma reesei* (Figur 9) drei Signale des Spektrums den Proteinen Hydrophobin I und II beziehungsweise einem Fragment von Hydrophobin II zugeordnet werden. Diese identifizierten Signale S_{id} können sowohl zur Abgrenzung gegenüber Bakterien, die keine Hydrophobine besitzen, als auch zur Differenzierung verschiedener Pilze untereinander herangezogen werden.

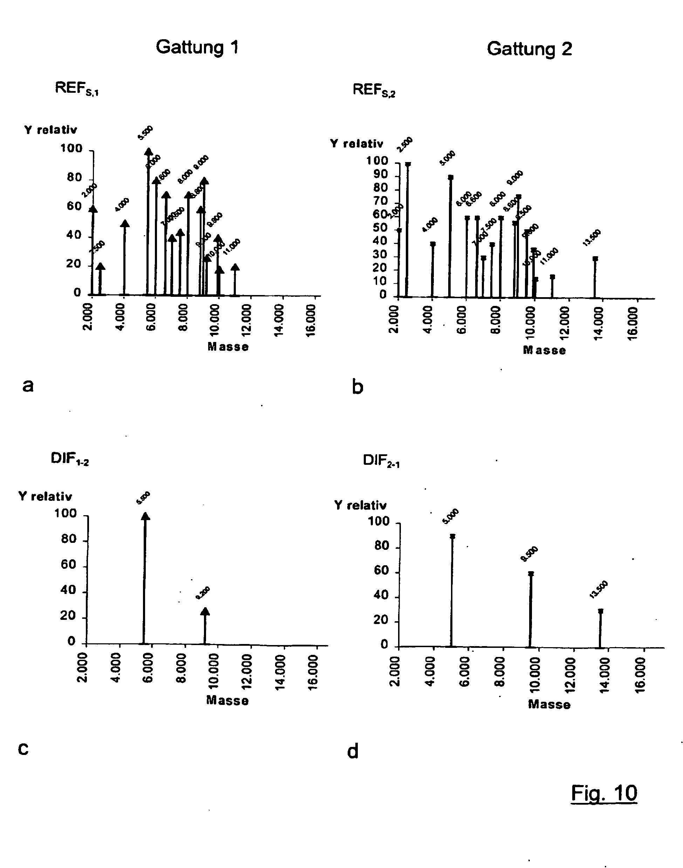

Weiter in Figur 1 werden anschließend an die Erzeugung der synthetischen Referenzspektren REF_{S} in dem Schritt S5 durch Verrechnung jeweils zweier Referenzspektren REF_{S} Differenzspektren DIF erzeugt. Dieser Schritt ist in den Figuren 10a bis 10d anhand von fiktiven synthetischen Referenzspektren zweier eng verwandter Organismen, hier der Gattung 1 und der Gattung 2 (Figuren 10a und 10b), verdeutlicht. Vorzugsweise wird dieser Schritt nur für einander sehr ähnliche Organismen durchgeführt, wobei die resultierenden Differenzspektren DIF die Unterscheidung genau dieser Organismen erleichtern.

Die Verrechnung der Referenzspektren REF_{S} erfolgt durch Subtraktion eines Referenzspektrums REF_{S} von dem jeweils anderen, wobei ein Signal, welches in beiden Referenzspektren REF_{S} vorhanden ist, unabhängig von den Intensitäten eliminiert wird. Auf diese Weise verbleiben in dem Differenzspektrum DIF₁₋₂ von Gattung 1 nur Signale, die nicht im Referenzspektrum REF_{S,2} von Gattung 2 vorhanden sind und umgekehrt. Figur 10c zeigt das um Gattung 2 "bereinigte" Differenzspektrum für Gattung 1 DIF₁₋₂ und Figur 10d das Differenzspektrum für Gattung 2 DIF₂₋₁ nach "Subtraktion" von Gattung 1. Selbstverständlich müssen bei der Verrechnung zweier Signale etwaige geräte- und messspezifische Toleranzen der Massen berücksichtigt werden, so dass im Ergebnis auch Signale S entfernt werden, die sich etwa um eine Massenzahl unterscheiden.

Signale, die nach der Subtraktion in den jeweiligen Differenzspektren DIF₁₋₂, DIF₂₋₁ verbleiben, können einer weiteren Selektion unterworfen werden. Beispielsweise kann für das Spektrum DIF₁₋₂ überprüft werden, wie häufig das Signal bei der Masse 5.500 in den natürlichen Original spektren von Gattung 1 (REF_{N,1}, nicht dargestellt) auftaucht und wie häufig in den Original spektren von Gattung 2 (REF_{N,2}, nicht dargestellt). Dabei wird ein Signal um so eher eliminiert, je seltener es in den natürlichen Eigenspektren und je häufiger in den natürlichen Fremdspektren auftaucht, wobei auch Intensitäten entsprechend berücksichtigt werden können.

Die in Schritt S4 (Figur 1) erzeugten synthetischen Referenzspektren REF_{S} sowie die in Schritt S5 erzeugten Differenzspektren DIF werden in einem anschließenden Schritt S6 zu einer Datenbank DB zusammengefasst. Innerhalb der Datenbank DB können die synthetischen Referenzspektren REF_{S} in logischer Weise, beispielsweise nach Familie, Gattung, Art und Stamm, geordnet werden. Ferner kann die Datenbank DB eine Statistik umfassen, die wiedergibt, wie sich ein Signal S bei der Erstellung der synthetischen Referenzspektren REF_{S} und/oder den Differenzspektren DIF hinsichtlich Häufigkeit und Spezifität bewährt hat. Diese Statistik kann beispielsweise der Gewichtung der Signale S bei der Identifizierung unbekannter Organismen dienen.

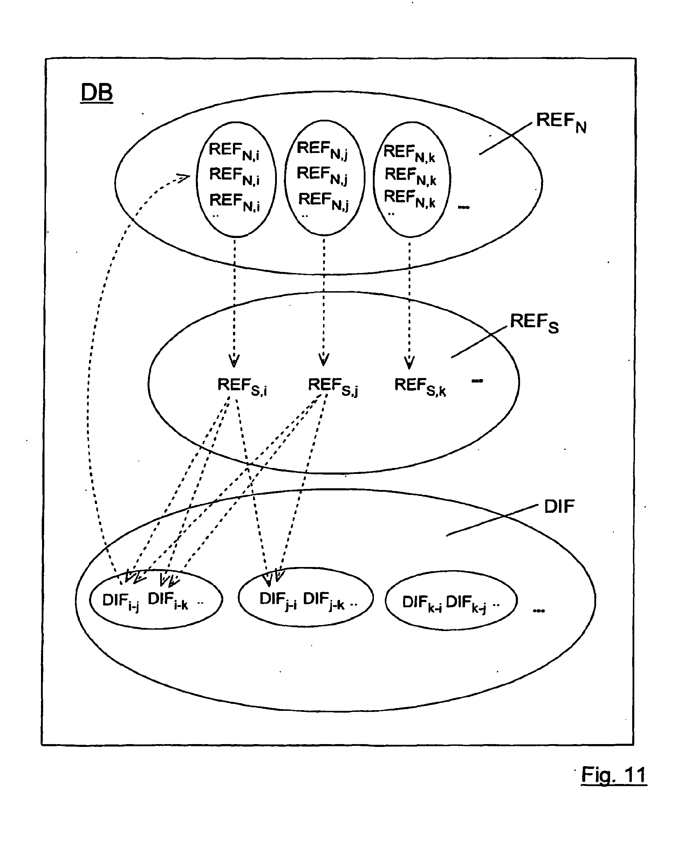

Der Aufbau der erfindungsgemäßen Datenbank DB ist in Figur 11 dargestellt. Die Datenbank umfasst (optional) natürliche Referenzspektren REF_{N}, wobei für jeden Organismus i, j, k und so weiter eine Vielzahl von Referenzspektren REF_{N} vorhanden sind. Die Datenbank DB beinhaltet ferner für jeden bekannten Organismus aus den jeweiligen natürlichen Referenzspektren REF_{N} ermittelte synthetische Referenzspektren REF_{S}, die aus wenigen diskreten (identifizierten und/oder empirischen) Signalen S bestehen. Schließlich umfasst die Datenbank DB Differenzspektren DIF, die aus jeweils zwei synthetischen Referenzspektren REF_{S} zweier Mikroorganismen ermittelt wurden. Dabei hat sich als ausreichend erwiesen, nur Differenzspektren DIF von solchen Organismen zu erstellen, die einander stark ähneln und daher erfahrungsgemäß schwer massenspektrometrisch zu unterscheiden sind.

Der Verfahrensablauf von S1 bis S6 gemäß Figur 1, das heißt die Erstellung der Datenbank DB, muss prinzipiell nur einmal durchgeführt werden. Auf diese Datenbank DB kann dann in dem erfindungsgemäßen Identifizierungsprozess immer wieder zurückgegriffen werden. Selbstverständlich kann und sollte die Datenbank DB aber ständig durch Referenzspektren REF_{S} und/oder Differenzspektren DIF weiterer Mikroorganismen erweitert und aktualisiert werden. So ist etwa denkbar, Referenzspektren REF_{S} von Mutanten, insbesondere solchen mit verändertem Resistenz- und/oder Virulenzverhalten, aufzunehmen, sofern diese Stämme charakteristische Signale liefern. Auch sollten auf Basis statistischer Auswertung der analysierten Proben die vorhandenen Referenzspektren REF_{S} laufend optimiert werden, beispielsweise durch die Aufnahme neuer Signale S_{id}, Sₑₘ und/oder durch Löschung von Signalen.

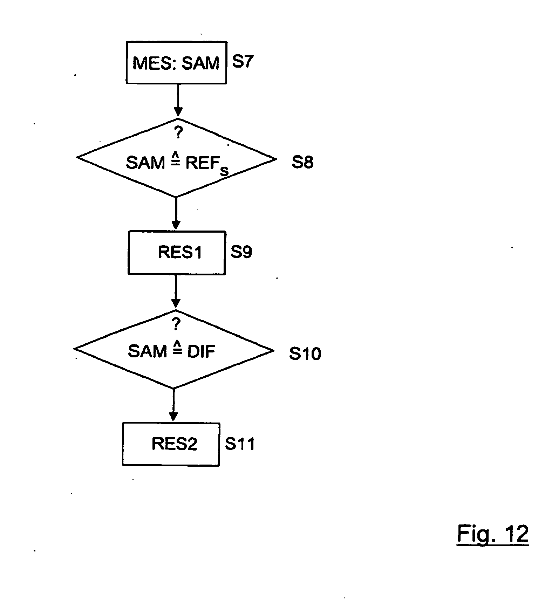

Der Ablauf des eigentlichen Identifizierungsverfahrens unter Verwendung der beschriebenen Datenbank DB ist in Figur 12 erläutert. In dem Schritt S7 erfolgt die Akquisition eines Massenspektrums SAM eines zu identifizierenden Mikroorganismus. Vorteilhafterweise wird dieses Probenspektrum SAM unter möglichst identischen Messbedingungen, insbesondere gleicher Matrix, wie die natürlichen Referenzspektren REF_{N} gemessen.

Nachfolgend (Schritt S8) erfolgt in einem ersten Analyseschritt eine Ähnlichkeitsanalyse des Probenspektrums SAM mit den in der Datenbank DB enthaltenen synthetischen Referenzspektren REF_{S}, wobei das Probenspektrum mit jedem Referenzspektrum REF_{S} verglichen wird. Dieser Spektrenabgleich kann visuell nach bestimmten vorgegebenen Kriterien, vorzugsweise jedoch computergestützt, erfolgen. Dabei kann prinzipiell auf die gleichen oder ähnliche Algorithmen zurückgegriffen werden, die bereits in Schritt S3 zur Ermittlung der empirischen Signale Sₑₘ Anwendung fanden. Durch die Einfachheit der verwendeten Referenzspektren REF_{S} kann der Spektrenabgleich in Schritt S8 auch mit sehr einfachen Algorithmen durchgeführt werden, die sich lediglich auf einen Vergleich der Signale S_{id,} Sₑₘ der synthetischen Referenzspektren REF_{S} mit dem Probenspektrum SAM beschränken. Selbstverständlich müssen hier Toleranzkriterien vorgegeben werden, die festlegen, um wie viel ein in Frage kommendes Signal S_{id}, Sₑₘ des Probenspektrums SAM hinsichtlich der Masse und/oder der Intensität abweichen darf, um als übereinstimmend gewertet zu werden. Außerdem können an dieser Stelle die in der Statistik enthaltenen Gewichtungen der einzelnen Signale S einfließen, wobei ein Signal S umso entscheidender für eine positive Identifizierung ist je öfter und intensiver es in den natürlichen Eigenspektren und je seltener und schwächer es in den natürlichen Fremdspektren vorhanden ist. Ferner kann eine Mindest-Anzahl übereinstimmender Signale für eine positive Identifizierung vorgegeben werden.

Im Schritt S9 erfolgt eine Ausgabe eines ersten Resultats RES1. Dieses kann entweder darin bestehen, dass bereits eine eindeutige Übereinstimmung des Probenspektrums SAM mit einem der Referenzspektren REF_{S} erkannt wurde, mit der Folge, dass das Verfahren an dieser Stelle bereits beendet ist. Weist das Probenspektrum SAM hingegen Ähnlichkeit mit mehr als einem synthetischen Referenzspektrum REF_{S} auf, so wird als Ergebnis REF1 eine Anzahl der in Frage kommenden Mikroorganismen ausgegeben. In diesem Fall schließt in Schritt S10 als zweiter Analyseschritt eine Ähnlichkeitsanalyse des Probenspektrums SAM mit in der Datenbank DB enthaltenen Differenzspektren DIF an. Wird in Schritt S8 beispielsweise eine hohe Ähnlichkeit mit den Referenzspektren REF_{S} der Organismen i und k festgestellt, so erfolgt in Schritt S10 ein Abgleich des Probenspektrums SAM mit den zwischen diesen Organismen erzeugten Differenzspektren DIFᵢ₋ₖ und DIFₖ₋ᵢ (vergleiche Figur 11). Der erfindungsgemäße zweite Analyseschritt erlaubt somit eine sichere Zuordnung eines unbekannten Organismus zu einem Organismus aus einer Gruppe einander sehr ähnlicher Organismen.

Als Endergebnis RES2 wird in Schritt S11 der mit dem Probenspektrum SAM als übereinstimmend ermittelte Mikroorganismus ausgegeben. Ferner kann auch eine Information über den Grad der Übereinstimmung ausgegeben werden. Handelt es sich - wie häufig der Fall - bei der Probe des unbekannten Organismus um eine Mischkultur, so ermöglicht das Verfahren sogar die Identifizierung mehrerer, nebeneinander vorliegender Mikroorganismen. In einem solchen Fall werden als Ergebnis mehrere Treffer ausgegeben. Stimmt das Probenspektrum SAM mit keinem der synthetischen Referenzspektren REF_{S} innerhalb der zugelassenen Toleranzen überein, das heißt, der unbekannte Mikroorganismus ist nicht in der Datenbank DB enthalten, so ist auch dieses ein ausgebbares Resultat.

In bestimmten Fällen, in denen ein Mikroorganismus nicht oder nicht eindeutig identifiziert werden kann, kann vorteilhaft vorgesehen sein, dass im Anschluss an den Schritt S11 ein zusätzlicher Direktvergleich mit den gemessenen "natürlichen" Referenzspektren REF_{N} durchgeführt wird. Dieses Vorgehen entspricht der bekannten Vorgehensweise zur Identifizierung von Mikroorganismen mittels MALDI-TOF-MS und erfordert eine Datenbank DB, die neben den Synthetischen Referenzspektren REF_{S} und den Differenzspektren DIF auch die natürlichen Referenzspektren REF_{N} umfasst.

Durch die Einfachheit und den konzentrierten Informationsgehalt der synthetischen Referenzspektren REF_{S} zeichnet sich das erfindungsgemäße gegenüber bekannten Verfahren durch eine wesentlich höhere Zuverlässigkeit aus, die durch den zweiten Analyseschritt noch weiter erhöht wird.

### Bezugszeichenliste

- S1: Messung von Referenzspektren
- S2: Signalidentifizierung
- S3: Ermittlung empirischer Signale
- S4: Erzeugung eines synthetischen Referenzspektrums
- S5: Erzeugung von Differenzspektren
- S6: Datenbankerstellung
- S7: Erfassung eines Probenspektrums
- S8: Abgleich: Probenspektrum/synthetische Referenzspektren
- S9: Ergebnisausgabe 1
- S10: Abgleich: Probenspektrum/Differenzspektren
- S11: Ergebnisausgabe 2

- DB: Datenbank
- DIF: Differenzspektrum
- REF_{N}: natürliches Referenzspektrum
- REF_{S}: synthetisches Referenzspektrum
- RES1: Ergebnis des ersten Analyseschrittes
- RES2: Ergebnis des zweiten Analyseschrittes
- SAM: Probenspektrum
- S_{id}: identifiziertes Signal
- Sₑₘ: empirisches Signal

## Patentansprüche

1. Verfahren zur Identifizierung von Mikroorganismen mittels Massenspektrometrie, wobei
(a) eine Datenbank (DB) verwendet wird, umfassend
- synthetische Referenzspektren (REF_{S}) bekannter Mikroorganismen, welche durch Zusammenfassung einer gegenüber natürlichen Massenspektren (REF_{N}) reduzierten Anzahl von für den jeweiligen Mikroorganismus spezifischen Signalen (S) gebildet werden, sowie
- Differenzspektren (DIF), welche durch Verrechnung jeweils zweier synthetischer Referenzspektren (REF_{S}) der bekannten Mikroorganismen gebildet werden,
(b) in einem ersten Analyseschritt eine Ähnlichkeitsanalyse eines Probenmassenspektrums (SAM) eines zu identifizierenden Mikroorganismus mit den in der Datenbank (DB) enthaltenen synthetischen Referenzspektren (REF_{S}) durchgeführt wird und
(c) in einem zweiten Analyseschritt eine Ähnlichkeitsanalyse des Probenspektrums (SAM) mit wenigstens einem Teil der in der Datenbank (DB) enthaltenen Differenzspektren (DIF) durchgeführt wird.

2. Verfahren nach Anspruch 1, **dadurch gekennzeichnet, dass** die Differenzspektren (DIF) durch Subtraktion zweier synthetischer Referenzspektren (REF_{S}) gebildet werden derart, dass Signale (S), die in beiden der miteinander verrechneten Referenzspektren (REF_{S}) vorhanden sind, intensitätsunabhängig eliminiert werden.

3. Verfahren nach Anspruch 2, **dadurch gekennzeichnet, dass** Signale (S), die nach der Subtraktion in einem Differenzspektrum (DIF) eines bekannten Mikroorganismus verbleiben, durch Abgleich mit den natürlichen Massenspektren (REF_{N}) dieses Mikroorganismus und/oder mit den natürlichen Massenspektren (REF_{N}) des mit diesem verrechneten Mikroorganismus selektiert und/oder gewichtet werden.

4. Verfahren nach Anspruch 3, **dadurch gekennzeichnet, dass** die Selektion und/oder Gewichtung der Signale (S) des Differenzspektrums (DIF) in Abhängigkeit ihrer Häufigkeit und/oder Intensität in den natürlichen Massenspektren (REF_{N}) des betreffenden Mikroorganismus und/oder in Abhängigkeit ihrer Häufigkeit und/oder Intensität in den natürlichen Massenspektren (REF_{N}) des mit diesem Mikroorganismus verrechneten Mikroorganismus erfolgt.

5. Verfahren nach Anspruch 1, **dadurch gekennzeichnet, dass** als Ergebnis des ersten Analyseschrittes eine Anzahl von mit dem Probenspektrum (SAM) ähnlichen synthetischen Referenzspektren (REF_{S}) ermittelt wird und die Ähnlichkeitsanalyse des zweiten Schrittes nur mit denjenigen Differenzspektren (DIF) durchgeführt wird, die aus der Verrechnung der als ähnlich ermittelten synthetischen Referenzspektren (REF_{S}) erhalten wurden.

6. Verfahren nach Anspruch 5, **dadurch gekennzeichnet, dass** die Datenbank (DB) nur Differenzspektren (DIF) von Mikroorganismen umfasst, die einander ähnelnde synthetische Referenzspektren (REF_{S}) aufweisen.

7. Verfahren nach einem der vorhergehenden Ansprüche, **dadurch gekennzeichnet, dass** die Signale (S) eines synthetischen Referenzspektrums (REF_{S}) mindestens ein identifiziertes Signal (S_{id}) umfassen, welches einem bestimmten molekularen Zellbestandteil des jeweiligen Mikroorganismus zugeordnet wurde.

8. Verfahren nach Anspruch 7, **dadurch gekennzeichnet, dass** der Zellbestandteil ein Peptid, ein Protein, eine Ribonukleinsäure und/oder ein Lipid ist.

9. Verfahren nach einem der Ansprüche 7 oder 8, **dadurch gekennzeichnet, dass** der Mikroorganismus ein Bakterium und der Zellbestandteil ein ribosomales Protein ist.

10. Verfahren nach einem der Ansprüche 7 oder 8, **dadurch gekennzeichnet, dass** der Mikroorganismus ein Pilz und der Zellbestandteil ein Strukturprotein, insbesondere ein Hydrophobin, und/oder ein ribosomales Protein ist.

11. Verfahren nach einem der vorhergehenden Ansprüche, **dadurch gekennzeichnet, dass** die Signale (S) eines synthetischen Referenzspektrums (REF_{S}) mindestens ein empirisches Signal (Sₑₘ) umfassen, welches durch Vergleich von Massenspektren bekannter Mikroorganismen als spezifisch für einen Mikroorganismus ermittelt wurde.

12. Verfahren nach Anspruch 11, **dadurch gekennzeichnet, dass** der Vergleich visuell und/oder computergestützt durchgeführt wird.

13. Verfahren nach einem der Ansprüche 11 oder 12, **dadurch gekennzeichnet, dass** als Kriterien zur Ermittlung eines empirischen Signals (Sₑₘ) eine vorgebbare Mindest-Häufigkeit für ein Auftreten des Signals in einer Anzahl von Massenspektren desselben Mikroorganismus und eine vorgebbare Mindest-Intensität des Signals vorgegeben werden.

14. Verfahren nach einem der vorhergehenden Ansprüche, **dadurch gekennzeichnet, dass** ein Signal (S) durch ein Koordinatenpaar, bestehend aus einer Masse (m) oder einem Masse-Ladungsverhältnis (m/z) als x-Koordinate und einer absoluten oder relativen Intensität als y-Koordinate, repräsentiert wird.

15. Verfahren nach einem der vorhergehenden Ansprüche, **dadurch gekennzeichnet, dass** die Anzahl der Signale (S) eines synthetischen Referenzspektrums (REF_{S}) 1 bis 50, insbesondere 5 bis 30, beträgt.

16. Verfahren nach einem der vorhergehenden Ansprüche, **dadurch gekennzeichnet, dass** die Aufnahme der Massenspektren von nicht vorbehandelten Zellen durchgeführt wird.

17. Verfahren nach einem der vorhergehenden Ansprüche, **dadurch gekennzeichnet, dass** die Ähnlichkeitsanalyse des Massenspektrums (SAM) des zu identifizierenden Mikroorganismus mit den in der Datenbank (DB) enthaltenen Referenzspektren (REF_{S}) sich auf einen Vergleich der in den Referenzspektren (REF) enthaltenen Signale (S) beschränkt.

18. Verfahren nach einem der vorhergehenden Ansprüche, **dadurch gekennzeichnet, dass** die Ähnlichkeitsanalyse des Massenspektrums (SAM) des zu identifizierenden Mikroorganismus mit den in der Datenbank (DB) enthaltenen Differenzspektren (DIF) sich auf einen Vergleich der in den Differenzspektren (DIF) enthaltenen Signale (S) beschränkt.

19. Verfahren nach einem der vorhergehenden Ansprüche, **dadurch gekennzeichnet, dass** für die Ähnlichkeitsanalyse den in den Referenzspektren (REF_{S}) enthaltenen Signalen (S) Gewichtungen zugeordnet werden.

20. Verfahren nach einem der vorhergehenden Ansprüche, **dadurch gekennzeichnet, dass** alle Massenspektren mit MALDI-TOF-MS aufgenommen werden.

## Claims

1. A method for identifying micro-organisms by means of mass spectrometry wherein
(a) a database (DB) is used, comprising
- synthetic reference spectra (REF_{S}) of known micro-organisms which are obtained by assembling a number of signals (S) specific to the respective micro-organism, which number is reduced compared to natural mass spectra (REF_{N}), and
- difference spectra (DIF) which are obtained by offsetting two synthetic reference spectra (REF_{S}) of the known micro-organisms against one another in each case,
(b) in a first analysis step, a similarity analysis of a sample mass spectrum (SAM) of a micro-organism to be identified with the synthetic reference spectra (REF_{S}) contained in the database (DB) is carried out, and
(c) in a second analysis step, a similarity analysis of the sample spectrum (SAM) with at least a part of the difference spectra (DIF) contained in the database (DB) is carried out.

2. A method according to claim 1, **characterized in that** the difference spectra (DIF) are obtained by subtracting two synthetic reference spectra (REF_{S}), such that signals (S) present in both reference spectra (REF_{S}) to be offset against one another are eliminated independent of their intensity.

3. A method according to claim 2, **characterized in that** signals (S) which remain within a difference spectrum (DIF) of a known micro-organism after the subtraction are selected and/or weighted by comparing them with the natural mass spectra (REF_{N}) of this micro-organism and/or with the natural mass spectra (REF_{N}) of the micro-organism offset against this micro-organism.

4. A method according to claim 3, **characterized in that** the selection and/or weighting of the signals (S) of the difference spectrum (DIF) is carried out depending on their frequency and/or intensity within the natural mass spectra (REF_{N}) of the micro-organism in question and/or depending on their frequency and/or intensity in the natural mass spectra (REF_{N}) of the micro-organism offset against this micro-organism.

5. A method according to claim 1, **characterized in that** as a result of the first analysis step, a number of synthetic reference spectra (REF_{S}) is identified which are similar to the sample spectrum (SAM), and the similarity analysis of the second step is carried out using only those difference spectra (DIF) obtained by offsetting the synthetic reference spectra (REF_{S}) which were found to be similar.

6. A method according to claim 5, **characterized in that** the database (DB) comprises only difference spectra (DIF) of micro-organisms having similar synthetic reference spectra (REF_{S}).

7. A method according to any one of the preceding claims, **characterized in that** the signals (S) of a synthetic reference spectrum (REF_{S}) comprise at least one identified signal (S_{id}) which has been assigned to a specific molecular cellular constituent of the respective micro-organism.

8. A method according to claim 7, **characterized in that** the cellular constituent is a peptide, a protein, a ribonucleic acid and/or a lipid.

9. A method according to claim 7 or 8, **characterized in that** the micro-organism is a bacterium and the cellular constituent is a ribosomal protein.

10. A method according to claim 7 or 8, **characterized in that** the micro-organism is a fungus and the cellular constituent is a structural protein, particularly a hydrophobin, and/or a ribosomal protein.

11. A method according to any one of the preceding claims, **characterized in that** the signals (S) of a synthetic reference spectrum (REF_{S}) comprise at least one empirical signal (Sₑₘ) which was found to be specific to a micro-organism by comparing mass spectra of known micro-organisms.

12. A method according to claim 11, **characterized in that** the comparison is carried out visually and/or computer-based.

13. A method according to claim 11 or 12, **characterized in that** a predeterminable minimum frequency for an occurrence of the signal in a number of mass spectra of the same micro-organism and a predeterminable minimum intensity of the signal are predetermined as the criteria to identify an empirical signal (Sₑₘ).

14. A method according to any one of the preceding claims, **characterized in that** a signal (S) is represented by a pair of coordinates consisting of a mass (m) or a mass/charge ratio (m/z) as the x coordinate and an absolute or relative intensity as the y coordinate.

15. A method according to any one of the preceding claims, **characterized in that** the number of signals (S) of a synthetic reference spectrum (REF_{S}) is 1 to 50, particularly 5 to 30.

16. A method according to any one of the preceding claims, **characterized in that** the mass spectra are acquired using non-pretreated cells.

17. A method according to any one of the preceding claims, **characterized in that** the similarity analysis of the mass spectrum (SAM) of the micro-organism to be identified with the reference spectra (REF_{S}) contained in the database (DB) is limited to a comparison of the signals (S) contained within the reference spectra (REF).

18. A method according to any one of the preceding claims, **characterized in that** the similarity analysis of the mass spectrum (SAM) of the micro-organism to be identified with the difference spectra (DIF) contained in the database (DB) is limited to a comparison of the signals (S) contained within the difference spectra (DIF).

19. A method according to any one of the preceding claims, **characterized in that** weights are assigned to the signals (S) contained within the reference spectra (REF_{S}) for the purpose of the similarity analysis.

20. A method according to any one of the preceding claims, **characterized in that** all mass spectra are acquired using MALDI-TOF-MS.

## Revendications

1. Méthode d'identification de microorganismes par la spectrométrie de masse,
(a) une base de donnés (DB) étant utilisée, comprenant
- des spectres de référence synthétiques (REF_{S}) de microorganismes connus qui sont formés par la réunion d'un nombre, réduit par rapport aux spectres de masse naturels (REF_{N}), de signaux (S) spécifiques au microorganisme respectif, ainsi que
- des spectres de différence (DIF) qui sont formés par la compensation à chaque fois de deux spectres de référence synthétiques (REF_{S}) des microorganismes connus,
(b) une analyse de similarité d'un spectre de masse d'échantillon (SAM) d'un microorganisme à identifier étant, dans une première étape d'analyse, effectuée avec les spectres de référence synthétiques (REF_{S}) contenus dans la base de données (DB), et
(c) une analyse de similarité du spectre d'échantillon (SAM) étant, dans une deuxième étape d'analyse, effectuée avec au moins une partie des spectres de différence (DIF) contenus dans la base de données (DB).

2. Méthode selon la revendication 1, **caractérisée en ce que** les spectres de différence (DIF) sont formés par soustraction de deux spectres de référence synthétiques (REF_{S}) de sorte que des signaux (S) qui sont présents dans deux des spectres de référence (REF_{S}) compensés l'un avec l'autre sont éliminés indépendamment de l'intensité.

3. Méthode selon la revendication 2, **caractérisée en ce que** des signaux (S) qui demeurent après la soustraction dans un spectre de différence (DIF) d'un microorganisme connu sont sélectionnés et/ou pondérés par comparaison aux spectres de masses naturels (REF_{N}) de ce microorganisme et/ou avec les spectres de masses naturels (REF_{N}) du microorganisme compensé avec celui-ci.

4. Méthode selon la revendication 3, **caractérisée en ce que** la sélection et/ou la pondération des signaux (S) du spectre de différence (DIF) a lieux en fonction de leur fréquence et/ou de leur intensité dans les spectres de masses naturels (REF_{N}) du microorganisme concerné et/ou en fonction de leur fréquence et/ou de leur intensité dans les spectres de masses naturels (REF_{N}) du microorganisme compensé avec ce microorganisme.

5. Méthode selon la revendication 1, **caractérisée en ce que,** comme résultat de la première étape d'analyse, un nombre de spectres de référence synthétiques (REF_{S}) similaires au spectre d'échantillon (SAM) est déterminé, et l'analyse de similarité de la deuxième étape n'est effectuée qu'avec les spectres de différence (DIF) qui ont été obtenus à partir de la compensation des spectres de référence synthétiques (REF_{N}) déterminés comme similaires.

6. Méthode selon la revendication 5, **caractérisée en ce que** la base de données (DB) ne comprend que des spectres de différence (DIF) de microorganismes qui présentent des spectres de référence synthétiques (REF_{S}) qui se ressemblent.

7. Méthode selon l'une quelconque des revendications précédentes, **caractérisée en ce que** les signaux (S) d'un spectre de référence synthétique (REF_{S}) comprennent au moins un signal identifié (S_{id}) qui a été affecté à un composant cellulaire moléculaire défini du microorganisme respectif.

8. Méthode selon la revendication 7, **caractérisée en ce que** le composant cellulaire est un peptide, une protéine, un acide ribonucléique et/ou un lipide.

9. Méthode selon l'une des revendications 7 ou 8, **caractérisée en ce que** le microorganisme est une bactérie, et le composant cellulaire une protéine ribosomale.

10. Méthode selon l'une des revendications 7 ou 8, **caractérisée en ce que** le microorganisme est un champignon et le composant cellulaire une protéine structurelle, en particulier une protéine hydrophobine, et/ou une protéine ribosomale.

11. Méthode selon l'une quelconque des revendications précédentes, **caractérisée en ce que** les signaux (S) d'un spectre de référence synthétique (REF_{S}) comprennent au moins un signal empirique (Sₑₘ) qui a été déterminé comme spécifique à un microorganisme par la comparaison de spectres de masse de microorganismes connus.

12. Méthode selon la revendication 11, **caractérisée en ce que** la comparaison est effectuée visuellement et/ou avec l'aide d'un ordinateur.

13. Méthode selon l'une des revendications 11 ou 12, **caractérisée en ce qu'**une fréquence minimale paramétrable d'apparition du signal dans un certain nombre de spectres de masse du même microorganisme et une intensité minimale paramétrable du signal sont paramétrées comme critères de détermination d'un signal empirique (Sₑₘ).

14. Méthode selon l'une quelconque des revendications précédentes, **caractérisée en ce qu'**un signal (S) est représenté par une paire de coordonnées, composée d'une masse (m) ou d'un rapport masse-charge (m/z) en tant que coordonnée x, et d'une intensité absolue ou relative en tant que coordonnée y.

15. Méthode selon l'une quelconque des revendications précédentes, **caractérisée en ce que** le nombre de signaux (S) d'un spectre de référence synthétique (REF_{S}) est de 1 à 50, de préférence de 5 à 30.

16. Méthode selon l'une quelconque des revendications précédentes, **caractérisée en ce que** l'enregistrement des spectres de masse est effectué par des cellules non prétraitées.

17. Méthode selon l'une quelconque des revendications précédentes, **caractérisée en ce que** l'analyse de similarité du spectre de masse (SAM) du microorganisme à identifier avec les spectres de référence synthétiques (REF_{S}) contenus dans la base de données (DB) se limite à une comparaison des signaux (S) contenus dans les spectres de référence (REF).

18. Méthode selon l'une quelconque des revendications précédentes, **caractérisée en ce que** l'analyse de similarité du spectre de masse (SAM) du microorganisme à identifier avec les spectres de différence (DIF) contenus dans la base de données (DB) se limite à une comparaison des signaux (S) contenus dans les spectres de différence (DIF).

19. Méthode selon l'une quelconque des revendications précédentes, **caractérisée en ce que,** pour l'analyse de similarité, des pondérations sont affectées aux signaux (S) contenus dans les spectres de référence (REF_{S}).

20. Méthode selon l'une quelconque des revendications précédentes, **caractérisée en ce que** tous les spectres de masse sont enregistrés avec MALDI-TOF-MS.
